(19) Europäisches Patentamt
European Patent Office
Office européen des brevets

(11) Veröffentlichungsnummer: 0 160 249
B1

(12) # EUROPÄISCHE PATENTSCHRIFT

(45) Veröffentlichungstag der Patentschrifft: 28.02.90

(51) Int. Cl.[5]: C 07 C 45/50, C 07 C 47/02

(21) Anmeldenummer: 85104606.0

(22) Anmeldetag: 16.04.85

(54) **Verfahren zur Herstellung von Aldehyden.**

(30) Priorität: 28.04.84 DE 3415968

(43) Veröffentlichungstag der Anmeldung: 06.11.85 Patentblatt 85/45

(45) Bekanntmachung des Hinweises auf die Patenterteilung: 28.02.90 Patentblatt 90/09

(84) Bennante Vertragsstaaten: DE FR GB IT NL SE

(56) Entgegenhaltungen:
DE-A-660 619
DE-A-2 045 169
FR-A-2 224 430
FR-A-2 314 910
FR-A-2 489 308
GB-A-1 086 100

(73) Patentinhaber: HOECHST AKTIENGESELLSCHAFT
Postfach 80 03 20
D-6230 Frankfurt am Main 80 (DE)

(72) Erfinder: Cornils, Boy, Dr. Dipl.-Chem.
Friedrich-Ebert-Strasse 45
D-4220 Dinslaken (DE)
Erfinder: Konkol, Werner, Dr. Dipl.-Chem.
Lützowstrasse 40
D-4200 Oberhausen 11 (DE)
Erfinder: Bach, Hanswilhelm, Dr. Dipl.-Chem.
Alleestrasse 56
D-4100 Duisburg 11 (DE)
Erfinder: Dämbkes, Georg, Dr. Dipl.-Chem.
Nibelungenstrasse 65
D-4220 Dinslaken (DE)
Erfinder: Gick, Wilhelm, Dr. Dipl.-Chem.
Im Buschhuck 8
D-4100 Duisburg 74 (DE)
Erfinder: Wiebus, Ernst
Ferdinandstrasse 77
D-4200 Oberhausen 1 (DE)
Erfinder: Bahrmann, Helmut, Dr. Dipl.-Chem.
Im Freihof 56
D-4224 Hünxe (DE)

EP 0 160 249 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

## Beschreibung

Die vorliegende Erfindung betrifft ein Verfahren zur Herstellung von Aldehyden durch Hydroformylierung von Olefinen in Gegenwart wasserlöslicher Rhodium-Komplexkatalysatoren mit dem Ziel, die Bildung höhersiedender Nebenprodukte zu reduzieren.

Es ist bekannt, durch Umsetzung von Olefinen mit Kohlenmonoxid und Wasserstoff Aldehyde und Alkohole herzustellen. Die Reaktion wird durch Hydridometallcarbonyle, vorzugsweise solcher der Metalle der 8. Gruppe des Periodensystems, katalysiert. Neben Kobalt, das als Katalysatormetall in großem Umfang technische Anwendung findet, gewinnt in letzer Zeit Rhodium zunehmende Bedeutung. Im Gegensatz zu Kobalt gestattet Rhodium, die Reaktion bei niedrigem Druck durchzuführen; darüber hinaus werden vorzugsweise geradkettige n-Aldehyde und nur in untergeordnetem Maße iso-Aldehyde gebildet. Schließlich ist auch die Hydrierung der Olefine zu gesättigten Kohlenwasserstoffen bei Anwendung von Rhodium-Katalysatoren deutlich niedriger als bei Anwendung von Kobalt-Katalysatoren.

Bei den in der Technik eingeführten Verfahren wird der Rhodium-Katalysator in Form von modifizierten Hydridorhodiumcarbonylen eingesetzt, die zusätzliche und gegebenenfalls überschüssige Liganden enthalten. Besonders bewährt haben sich als Liganden tertiäre Phosphine oder Phosphite. Ihre Anwendung ermöglicht, den Reaktionsdruck auf Werte unter 300 bar ($3 \cdot 10^4$ kPa) zu senken.

Probleme wirft bei diesem Verfahren jedoch die Abtrennung der Reaktionsprodukte und die Wiedergewinnung der im Reaktionsprodukt homogen gelösten Katalysatoren auf. Im allgemeinen destilliert man hierzu das Umsetzungsprodukt aus dem Reaktionsgemisch ab. In der Praxis kann dieser Weg wegen der thermischen Empfindlichkeit der gebildeten Aldehyde und Alkohole aber nur bei der Hydroformylierung niedriger Olefine, d.h. Olefinen mit bis zu etwa 5 Kohlenstoffatomen im Molekül, beschritten werden. Außerdem hat sich gezeigt, daß die thermische Belastung des Destillationsgutes auch zu erheblichen Katalysatorverlusten durch Zersetzung der Rhodiumkomplexverbindungen führt.

Die geschilderten Mängel werden durch Anwendung von Katalysatorsystemen vermieden, die in Wasser löslich sind. Derartige Katalysatoren sind z. B. in der DE-PS-2 627 354 beschrieben. Die Löslichkeit der Rhodiumkomplexverbindungen wird hierbei durch Verwendung von sulfonierten Triarylphosphinen als Komplexbestandteil erreicht. Die Abtrennung des Katalysators vom Reaktionsprodukt nach Beendigung der Hydroformylierungsreaktion erfolgt bei dieser Verfahrensvariante einfach durch Trennung von wäßriger und organischer Phase, d.h. ohne Destillation und damit ohne zusätzliche thermische Verfahrensschritte. Neben sulfonierten Triarylphosphinen werden als Komplexbestandteile wasserlöslicher Rhodiumkomplexverbindungen auch carboxylierte Triarylphosphine eingesetzt.

Ein weiterer Vorteil des unter Verwendung der neuen Katalysatoren durchgeführten Verfahrens ist die im Vergleich zu anderen Prozessen deutlich verminderte Bildung höhersiedender Nebenprodukte. Diese Verbindungen sind unerwünscht, denn sie entstehen aus den primär gebildeten Aldehyden und beeinträchtigen deshalb die Wertproduktausbeute.

Bekannte Verfahren zielen daher darauf ab, die höhersiedenden Verbindungen wieder in die Ausgangsaldehyde zurückzuspalten. So behandelt man nach der DE-PS-1 817 051 Produkte der Oxosynthese, die neben Aldehyden und Alkoholen noch Ester, polymere Aldehyde, ungesättigte Hydroxyaldehyde und Acetale enthalten bei 250 bis 350°C in Gegenwart Aluminiumoxid enthaltender Katalysatoren mit Wasser. Bei dieser Behandlung werden nicht nur die Ester, sondern auch die polymeren Aldehyde, die Aldole und die Acetale gespalten.

Zweckmäßiger als die Rückumwandlung von Nebenprodukten in Wertprodukte ist es, Maßnahmen zu ergreifen, die die Bildung von unerwünschten Verbindungen unterdrücken. Die klassischen Oxoverfahren mit Kobalt als Katalysator boten hierfür ebenso wenig Möglichkeiten, wie die modernen Oxoverfahren, die in homogener Phase mit wasserunlöslichen Rhodium-Phosphin-Komplexverbindungen als Katalysatoren arbeiten.

Überraschenderweise ist es möglich, bei Einsatz der vorstehend beschriebenen wasserlöslichen Rhodiumkomplexverbindungen als Katalysatoren auf einfachem Wege die an sich geringe Bildung von höhersiedenden Verbindungen noch weiter zurückzudrängen.

Die Erfindung besteht in einem Verfahren zur Herstellung von Aldehyden durch Umsetzung von aliphatischen Olefinen mit 2 bis 12 Kohlenstoffatomen mit Kohlenmonoxid und Wasserstoff in flüssiger Phase in Gegenwart von Wasser sowie von Rhodium in metallischer Form oder als Verbindung und dem wasserlöslichen Salz eines sulfonierten oder carboxylierten Triarylphosphins. Es ist dadurch gekennzeichnet, daß man dem Gemisch aus Kohlenmonoxid und Wasserstoff 0,5 bis 4,0 Vol. %, bezogen auf das gesamte Gasgemisch, Kohlendioxid zusetzt.

Aufgrund der bisherigen Kenntnisse über den Ablauf der Oxosynthese war es nicht vorauszusehen, daß der Zusatz von Kohlendioxid zum Reaktionsgemisch Einfluß auf die Produktzusammensetzung haben könnte. Im Gegenteil mußte erwartet werden, daß eine Desaktivierung des Katalysatorsystems eintritt, da Kohlendioxid in Gegenwart von Rhodium-Phosphin-Komplexverbindungen Phosphine zu Phosphinoxiden oxidiert (vgl. K.M. Nicholas, J. Organometal. Chem. 188 (1), C 10 (1980)

Kohlendioxid als Bestandteil des Reaktionsgemisches bewirkt, daß die Bildung von höhersiedenden Verbindungen um 50 bis 70 % reduziert wird. Bei niederen Olefinen, d. h. solchen mit bis zu 5 Kohlenstoffatomen ist die Verminderung des Nebenproduktanteils besonders ausgeprägt.

Kohlendioxid wird dem Reaktor in einer Menge von 0,5 bis 4,0 Vol. %, bezogen auf das Gemisch aus Wasserstoff, Kohlenmonoxid und Kohlendioxid, zugeführt. Geringere Kohlendioxid-Konzentrationen als angegeben beeinflussen die Nebenproduktbildung nicht mehr, höhere Konzentrationen führen zu einer Herabsetzung der Geschwindigkeit der Hydroformylierungsreaktion. Besonders bewährt hat es sich, den Kohlendioxidanteil auf 1 bis 3 Vol. % einzustellen.

Das Kohlendioxid kann dem Reaktor getrennt vom Kohlenmonoxid-Wasserstoff-Gemisch zugeführt werden. Zweckmäßiger ist es, die drei Gase gemeinsam in die Reaktionszone einzuleiten. In diesem Falle kann die erforderliche Kohlendioxid-Konzentration gegebenenfalls durch entsprechende Lenkung der zu CO und $H_2$ führenden Vergasungsreaktion oder durch gezielte Entfernung von Kohlendioxid aus dem $CO/H_2$-Gemisch eingestellt werden.

Nach dem erfindungsgemäßen Verfahren lassen sich Olefine mit 2 bis 12 Kohlenstoffatomen hydroformylieren. Diese Olefine können linear oder verzweigt sein mit einer endständigen oder innenständigen Doppelbindung. Beispiele für solche Olefine sind: Ethylen, Propylen, 1-Buten, 2-Buten, 1-Penten, 2-Methyl-1-buten, 1-Hexen, 2-Hexen, 1-Hepten, 1-Octen 3-Octen, 3-Ethyl-1-hexen, 1-Decen, 3-Undecen, 4,4-Dimethyl-1-nonen, 1-Dodecen. Bevorzugt werden lineare Olefine mit 2 bis 8 Kohlenstoffatomen wie Ethylen, Propylen, 1-Buten, 1-Penten, 1-Hexen, 1-Hepten und 1-Octen eingesetzt.

Als Katalysator findet Rhodium in metallischer Form oder in Form einer seiner Verbindungen zusammen mit einem wasserlöslichen Phosphin Anwendung, das der allgemeinen Formel

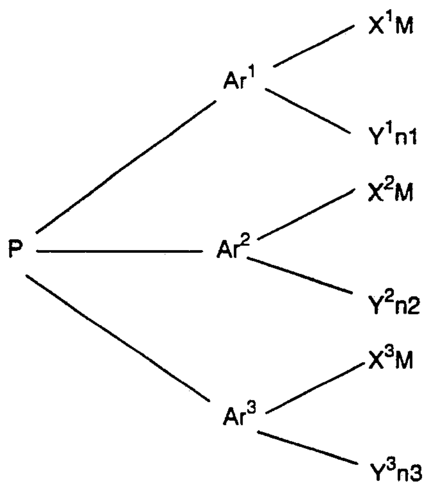

entspricht. Hierbei bedeuten $Ar^1$, $Ar^2$, $Ar^3$ jeweils eine Phenyl- oder Naphthylgruppe, $Y^1$, $Y^2$, $Y^3$ jeweils eine geradkettige oder verzweigte Alkylgruppe mit 1 bis 4 C-Atomen, eine Alkoxygrupe, ein Halogenatom, die OH-, CN-, $NO_2$- oder $R^1R^2N$-Gruppen, in der $R^1$ und $R^2$ für jeweils eine geradkettige oder verzweigte Alkylgruppe mit 1 bis 4 C-Atomen stehen; $X^1$, $X^2$, $X^3$ ist jeweils ein Carboxylat-(COO-) und/oder Sulfonat-($SO_3$-)Rest, $n_1$, $n_2$, $n_3$ sind gleiche oder verschiedene ganze Zahlen von 0 bis 5, M ist ein Alkalimetallion, das Äquivalent eines Erdalkalimetall- oder Zinkions oder ein Ammonium- oder quartäres Alkylammoniumion der allgemeinen Formel $N(R^3R^4R^5R^6)^+$ in der $R^3$, $R^4$, $R^5$, $R^6$ jeweils für eine geradkettige oder verzweigte Alkylgruppe mit 1 bis 4 C-Atomen steht.

Nach einer bevorzugten Ausführungsform dieses Verfahrens werden als wasserlösliche Phosphine Verbindungen der vorstehend beschriebenen allgemeinen Formel eingesetzt, in der $Ar^1$, $Ar^2$, $Ar^3$ jeweils eine Phenylrest und $X^1$, $X^2$, $X^3$ jeweils einen Sulfonatrest bedeuten. Beispiele für Verbindungen der oben wiedergegebenen allgemeinen Formel sind Triphenylphosphin-tri-Na-trisulfonat, Triphenylphosphin-tri-(tetraalkylammonium)-trisulfonat, Triphenylphosphin-tri-Na-tricarboxylat.

Das Rhodium wird entweder in metallischer Form oder als Verbindung eingesetzt. Metallisches Rhodium wird bevorzugt auf einem Träger wie Aktivkohle, Calciumcarbonat, Aluminiumsilikat, Tonerde aufgebracht, verwendet. Als Rhodiumverbindungen kommen solche Substanzen in Betracht, die wasserlöslich sind oder unter den Reaktionsbedingungen wasserlöslich werden. Geeignete Verbindungen sind die verschiedenen Rhodiumoxide, Salze anorganischer Wasserstoff- und Sauerstoffsäuren, sowie Salze aliphatischer Mono- und Polycarbonsäuren. Als Beispiele seien genannt Rhodiumchlorid, Rhodiumnitrat, Rhodiumsulfat, Rhodiumacetat, Rhodiummalonat. Weiterhin können Rhodiumcarbonylverbindungen wie Tricarbonylrhodium oder Tetracarbonylrhodium oder Komplexsalze des Rhodiums, z. B. Cyclooctadienylrhodiumchlorid eingesetzt werden. Bevorzugt werden Rhodiumoxid, Rhodiumchlorid und Rhodiumacetat.

Die Katalysatorlösung kann im voraus, z. B. aus der wässrigen Phosphinlösung und der benötigten Menge Rhodium, hergestellt und der Reaktionszone zugeführt werden. Es ist aber ebenso möglich, die Katalysatorlösung durch Vermischen der Komponenten in der Reaktionszone selbst herzustellen.

Die Rhodiumkonzentration in der wässrigen Katalysatorlösung beträgt vorzugsweise 10 bis 2000 Gew.-ppm, bezogen auf die Lösung. Das wasserlösliche Phosphin wird in einer solchen Menge eingesetzt, daß auf 1 Grammatom Rhodium 1 bis 1000 Mol, vorzugsweise 2 bis 300 Mol, Phosphinverbindung kommen.

Der Gesamtdruck von Wasserstoff und Kohlenmonoxid beträgt 1 bis 200 bar (100 bis $2 \cdot 10^4$ kPa), vorzugsweise 10 bis 100 bar ($1 \cdot 10^3$ bis $1 \cdot 10^4$ kPa). Die Zusammensetzung des Synthesegases, d. h. das Verhältnis von Kohlenmonoxid zu Wasserstoff, kann in weiten Grenzen variiert werden. Im allgemeinen setzt man ein Synthesegas ein, in dem das Volumverhältnis von Kohlenmonoxid zu Wasserstoff 1 : 1 beträgt oder von diesem Wert nur wenig abweicht. Die Umsetzung erfolgt bei Temperaturen von 20 bis 150°C, sie kann sowohl

kontinuierlich als auch absatzweise durchgeführt werden.
Die nachfolgende Beispiele erläutern die Erfindung.

**Beispiel** 1 (Vergleich)

In einem kontinuierlich betriebenen Rührkessel wird bei 120°C und einem Gesamtdruck von 25 bar ( $2{,}5 \cdot 10^3$ kPa) in Gegenwart von Rhodium und wässriger Triphenylphospbintrisulfonat-Lösung (500 Gew.-ppm Rh, bezogen auf die wässrige Lösung, molares Verhältnis Rhodium zu Phosphorverbindung: 1 : 80) soviel Propylen eingesetzt, daß entsprechend dem Olefinumsatz ein konstanter Partialdruck von Propylen, CO und $H_2$ im Verhältnis 2 : 1 : 1 eingehalten wird. Das Hydroformylierungs-Rohprodukt hat folgende Zusammensetzung:

| | |
|---|---|
| i-Butanal | 3,9 Gew.% |
| n-Butanal | 93,6 Gew.% |
| i-Butanol | 0,1 Gew.% |
| n-Butanol | 0,7 Gew.% |
| höhersiedende Verbindungen | 1,7 Gew.% |
| davon | |
| $C_8$-Aldole | 1,4 Gew.% |
| andere Höhersieder | 0,3 Gew.% |

**Beispiel 2**

Beispiel 1 wird mit dem Unterschied wiederholt, daß das Synthesegas 3 Vol. % Kohlendioxid (bezogen auf das Gesamtgewicht) zugemischt enthält. Bei gleicher Ausbeute hat das Hydroformylierungsprodukt folgende Zusammensetzung:

| | |
|---|---|
| i-Butanal | 3,9 Gew.% |
| n-Butanal | 94,7 Gew.% |
| i-Butanol | 0,1 Gew.% |
| n-Butanol | 0,8 Gew.% |
| höhersiedende Verbindungen | 0,5 Gew.% |
| davon | |
| $C_8$-Aldole | 0,2 Gew.% |
| andere Höhersieder | 0,3 Gew.% |

Die Überwachung der Katalysatorlösung über einen längeren Zeitraum ergibt, daß trotz der $CO_2$-Zugabe keine verstärkte Oxidation des Phosphins zu beobachten ist.

**Patentansprüche**

1. Verfahren zur Herstellung von Aldehyden durch Umsetzung von aliphatischen Olefinen mit 2 bis 12 Kohlenstoffatomen mit Kohlenmonoxid und Wasserstoff in flüssiger Phase in Gegenwart von Wasser sowie von Rhodium in metallischer Form oder als Verbindung und dem wasserlöslichen Salz eines sulfonierten oder carboxylierten Triarylphosphins, dadurch gekennzeichnet, daß dem Gemisch aus Kohlenmonoxid und Wasserstoff 0.5 bis 4,0 Vol. %, bezogen auf das gesamte Gasgemisch, Kohlendioxid zugesetzt werden.

2. Verfahren nach Anspruch 1, dadurch gekennzeichnet, daß dem Gemisch aus Kohlenmonoxid und Wasserstoff 1 bis 3 Vol. % bezogen auf das gesamte Gasgemisch Kohlendioxid zugesetzt werden.

**Claims**

1. A process for preparing aldehydes by the reaction of aliphatic olefins having 2 to 12 carbon atoms with carbon monoxide in liquid phase in the presence of water, rhodium in metallic form or as a compound and the water-soluble salt of a sulfonated or carboxylated triaryl phosphine, characterised in that 0.5 to 4.0 % by volume carbon dioxide, related to the total gas mixture, is added to the mixture of carbon monoxide and hydrogen.

2. A process according to claim 1, characterised in that 1.0 to 3.0 % by volume carbon dioxide, related to the total gas mixture, is added to the mixture of carbon monoxide and hydrogen.

**Revendications**

1. Procédé pour la fabrication d'aldéhydes par réaction d'oléfines aliphatiques en $C_2$ - $C_{12}$ avec le monoxyde de carbone et l'hydrogène en phase liquide en présence d'eau et de rhodium sous forme métallique ou de composé et d'un sel soluble dans l'eau d'une triarylphosphine sulfonée ou carboxylée, caractérisé en ce que l'on ajoute au mélange de monoxyde de carbone et d'hydrogène 0,5 à 4,0 % en volume de dioxyde de carbone, par rapport au mélange gazeux total.

2. Procédé selon la revendication 1, caractérisé en ce que l'on ajoute au mélange de monoxyde de carbone et d'hydrogène 1 à 3 % en volume de dioxyde de carbone par rapport au mélange gazeux total.